# EUROPEAN PATENT APPLICATION

(11) **EP 0 761 184 A2**
(43) Date of publication of application: **12.03.1997**
(21) Application number: 96305782.3
(22) Date of filing: 06.08.1996
(51) Int. Cl.: A61F 5/00

(54) **Improved straps for orthopaedic and orthotic appliances**

(30) Priority: 08.08.1995 GB 9516243
(71) Applicant: INNOVATIVE CARE LTD, Abingdon, Oxon OX14 5RY (GB)
(72) Inventor: Young, David Ernest, Watlington, Oxfordshire OX9 5QQ (GB)
(74) Representative: Crawford, Andrew Birkby

(57) **Abstract**

Strap means for an orthopaedic or orthotic appliance, the strap means being made from loop pile and hook pile strips and comprising: a first main portion (12) having a first end (24) provided with means for attachment to the appliance, said main portion having first and second iso-functional surfaces (18,22); and a second portion which comprises releasable and re-attachable connecting means (14) for securing said second portion to said main portion at a second end of said main portion, said second portion providing tab means for securing said second portion to a further area of said main portion of said strap during use of the appliance; the second portion having first and second elements (32,34) connected to each other by sewing across their widths to provide opposed iso-functional surfaces (36,38), said opposed surfaces of the first and second elements providing said connecting means for releasable re-attachment of the second portion to the second end of the main portion of the strap, wherein the width of said second portion is in the range 0.7 to 1.0 times the width of the main portion.

## Description

An improved strap for use with orthopaedic and orthotic appliances worn about the trunk and limbs is made from strips of hook and loop pile fabrics. The instant strap has a releasably reattachable end tab by means of which shortening and general adjustment of the strap is easier than with prior art straps and general versatility and security is enhanced.

### FIELD OF THE INVENTION

This invention relates to straps which are the means used for attaching and securing most orthoses and orthotic devices to the human body. In particular, the invention relates to novel means for allowing straps made from strips of hook and loop pile fabrics to be shortened, adjusted and re-positioned.

### BACKGROUND OF THE INVENTION

Narrow fabrics is a term used by those skilled in the art of textile manufacture to describe strip or ribbon materials such as those used to make textile straps. Hook pile and loop pile narrow fabrics, often referred to simply as 'hook and loop' have been used for strapping and closure purposes for many years and examples may be found in many industrial and domestic applications. These vary from the re-sealable edges of aircraft seat covers to simple tab closure patches on outdoor and infant clothing. Other examples include applications in the automotive industry such as securing trim panels and roof headlinings for the interiors of vehicles. In this disclosure I shall generally refer to lengths of such fabrics as strips, sections or portions.

In the fields of orthopaedics and orthotics strips of hook and loop pile fabrics are widely employed generally in 1'' (25mm) to 2'' (50mm) widths, particularly for the construction of straps. Such straps are employed for securing appliances, such as supports and braces, around or on to the trunk or a limb. Straps of this kind are sometimes called 'cinching' straps. This reflects both that they are used for tightening the appliance onto the limb and how such tightening is achieved. Typically, the main portion of the strap, made of loop pile material, is secured to the appliance at one end and the second end is then pawed through or around guide means. The guide means are secured to another area of the appliance and also provide restraining counterforce means. The strap is then pulled to the required tightness and finally it is secured to itself by apposition of an end tab usually formed by a strip of hook pile material attached to the swap body near the second end.

There are, however, a number of problems associated with the design and use of straps made from these materials. The tendency of hook sections of straps to catch on garments and other fabrics is widely recognised and any measure which reduced such a tendency without significantly reducing functionality would be broadly welcomed.

Another problem with straps of this type is maintaining security and reliability of the first attachment to the appliance during a protracted period of use. Many manufacturers stitch the first end of the main portion of the strap, which is almost always made of loop, directly to the appliance. Alternatively they employ some form of runner or guide, itself securely attached to the appliance and secure the first end of the strap to this. A variety of methods is employed in direct stitching which can vary from, at its simplest, a single line of stitching across the strap to a sewn pattern such as a rectangle and diagonals scheme. where the strap material and appliance material are suitable, high frequency or heat welding is sometimes employed, however, these process tend to degrade the materials and the weld strength is often unacceptably poor. when a runner is employed there is a third option for securing the first end of the strap. In this, a short hook section is attached to or over the first end of the main portion of the strap, by sewing or welding, in such a manner that the hook facing may be doubled around the runner and then apposed to the loop surface. Of course, in the less usual case where the main portion of the strap is made of hook pile fabric, the converse approach with a short section of loop is employed.

The second end of the main portion of a strap made of loop material usually has a section of hook material attached to or over it. This portion is often referred to by those skilled in the arts of orthotics and orthopaedics as the tab end or end tab. The end tab is secured to the main portion by sewing or welding and the same range of variations encountered in securing the first end is also seen here. There are no hard and fast rules for the free or functional lengths of end tabs in relation to the width of material from which they are made but in my experience they are rarely less than 3'' (75mm) - 4'' (100mm) long in the case of 1'' (25mm), 1½'' (38mm) and 2'' (50mm) widths. In addition, unless the end tab is fully oversewn onto the main portion of the strap, it requires an additional length allowance for overlapping attachment to the main portion. In sewn straps this is typically 1'' (25mm) to 1½'' (38mm) and in welded straps it is generally closer to ½'' (12.5mm). The welding method of attachment is used less and less frequently in straps over 1" (25mm) in width for orthopaedic and orthotic use because it is now widely recognised that the tractive loads likely to be imposed on such straps may well exceed the weld strength or the material strength in the weld zone.

I have not seen a strap where an end tab was formed by attaching free hook to free loop without sewing though I have used free hook lengths to secure simple brushed nylon (which is hook attachable) faced wraps and linings which themselves were to be retained and secured overall with full straps. In orthopaedic or orthotic practice it would probably be regarded as necessary to overlap the main portion of the strap in such an arrangement by 100% of the free length of the end tab. In other words, for a 3'' (75mm) length functional tab there would need to be a further 3'' (75mm) employed in an overlap.

This would not only be inconvenient but would render the strap unusable in many applications. For example in many adult men and women an end tab of overall length 6" (150mm) would easily exceed half the circumference of the forearm and in most it would exceed half the upper arm circumference.

A further problem with straps of this type relates to adjustment in their length, particularly in regard to shortening. It is unusual and rarely cost effective to make orthopaedic and orthotic appliances which are entirely customised and this is especially true in relation to straps. In most countries there is a general trend to employ ready made or off-the-shelf appliances made by commercial manufacturers. The manufacturer is confronted by the problem of having to decide upon a standardised range of sizes which will inevitably fit many patients less than perfectly. Making straps adjustable in length is an important way of achieving the best possible fit when patients have body measurements which do not correspond well with standard appliance sizes. For of example, in the field of braces for the lower limb, it is not unusual to encounter patients who have a mid-calf circumference which matches a manufacturer's large size recommendations yet have a thigh circumference which matches the small size recommendation. In such cases the discrepancy between the upper and lower leg measurements exceeds one full size. If a bespoke brace is not an option for economic or other reasons, a standard product would, of necessity, be used, quite possibly based on the largest dimension to be fitted. This inevitably means that some of the basic strap lengths would be in excess of what is needed and shortening of some straps would have to be undertaken at the time of filling the brace.

The most difficult case for the fitter is when a brace has to be used with one section known to be too large for the patient, where the first ends of the straps in that section are nonreleasably secured to it and the second ends are sewn or welded end tabs. It might appear that this problem is overcome or lessened where the first end is releasably secured to the main portion of the strap. The reasoning for this conclusion is that substantial shortening can be achieved by securing the first end of the main portion of such a strap not at the point nearest its runner but some distance away. However, this leads to a situation where multiple thicknesses of strap material are brought to bear in radial impingement against one part of the limb surface and the basic strap thickness bears against an adjacent area, leading to discomfort. The effect is worsened where double sided material (which is effectively double thickness) is used and worsened further in the zone where the co-functional releasable patch is attached. In such a case there will be five layers adjacent to two layers. In such cases, even when the strap is padded or lined, it is difficult to get patients to comply over an extended period. In any case, in order to make such adjustments, R is often necessary to take the brace off the leg, which is time consuming and inconvenient for both parties.

It may seem an attractive alternative to turn such a strap over so that the unequal layer problem is transferred to the outside and away from the patient's leg, however, this increases the tendency of straps to catch on the edges of chairs and car seats and is generally inconvenient when the patient is sitting. In fact, it is not an option with most prior art straps because the end tab is normally a single surface patch attached to or extending from one side of the second end of the main portion of the strap and turning over the straps renders them unusable.

One way of dealing with this problem has been to make the main strap section of single surface hook material and much longer than normal. In addition, a patch of loop material is sewn to or otherwise attached to both faces of the first, releasable, end. There is no separate end tab in such a strap. The long, hook, main portion is passed through the counterforce guide, cinched to the desired tension and then pressed against the exposed face of the loop at the first, releasable end. It may then be trimmed as required. The problem with this solution is that it is very wasteful of material and braces with several such straps can be difficult to handle due to the likelihood of entanglement.

### SUMMARY OF THE INVENTION

The present invention provides improvements to straps for orthopaedic and orthotic appliances used on the human body. In the invention the main portions of straps are made from strips of hook or loop pile fabrics in such a manner that at least part of these portions have two functional surfaces. A novel arrangement between the main portions of the straps and end tabs which are made from strips of co-functional hook or loop pile fabrics, provides quicker, easier and more economical adjustment and better patient comfort than prior art straps.

According to the present invention, there is provided at least one strap for virtually any orthosis or orthopaedic appliance which is to be secured about the human trunk or limbs. In the instant strap, a first structural element and main portion is made from a strip of loop pile or hook pile fabrics, attached releasably or non-releasably at a first end, directly or indirectly, to a first point on the appliance and which, at least in that part of the main portion likely to be required for adjustment, has first and second, parallel, outwards facing, iso-functional surfaces. The second end of the main portion of the strap passes through guide means, which also constitute restraining means, attached to a second point on the appliance and is doubled back on itself, such tractive load being applied to the strap against the restraining guide means as is appropriate for the purpose.

A second structural element of the strap is an end tab, releasably and re-attachably secured to both functional surfaces of the second end of the main portion of the strap. The end tab comprises a first, shorter, portion and second, longer, portion, both made from strips of hook pile or both made from loop pile fabrics, co-functional with the material selected for the main portion. The two portions of the end tab are secured together, non-releasably, preferably by secure sewing across their respective widths, with functional surfaces opposed (facing inwards) and aligned along their respective long axes. This is done in such a manner that they form an included angle, resembling a 'Y' in edge view, wherein the opposed functional surfaces of the included angle of the arms of the 'Y' provide releasable and re-attachable securing means for the end tab to the second end of the main portion of the strap. In addition, one or both surfaces of the stem of the 'Y' provide releasable securing means in the form of a free, functional tab end for releasably and re-attachably securing the end tab to the main portion of the strap. In use, the parallel and outwards facing functional surfaces of the second end of the main strap portion are inserted between the opposed inwards facing co-functional surfaces of the end tab which are then pressed together. The second end of the main portion of the strap is then passed through guide and counterforce means and doubled back on itself. A tractive load is applied to the strap and a functional surface of the stem of the end tab is then pressed against the corresponding surface of the main portion of the strap to secure it in place..

It is a major advantage of the present invention that, if shortening of the strap is necessary in order to fit the anatomy of a particular patient, the end tab may be removed quickly and easily, the main portion may be trimmed to length and the end tab re-attached. Alternatively, the end tab may be kept separate until a brief trial of the main portion strap length under tension is accomplished and any trimming deemed necessary is completed. This approach avoids the need for the fitter to have to remove the end tab during the fitting process and saves time for both parties. In a knee brace, for instance, there may be as many as six or even more straps, thus such savings can be very worthwhile. Furthermore, the strap of the instant invention allows adjustment, particularly shortening, to be carried out in all situations without removing the appliance from the patient. In addition, the main portions of straps according to the invention may be restricted to an economical length which makes handling easier and contains costs.

Since two opposed iso-functional surfaces are used in the releasable end tabs of the instant invention the lengths of each of them need not be substantially longer than the overlapped and sewn length of a conventional non-releasable end tab. This feature allows straps according to the present invention to be used in restricted areas, such as on small circumference limbs, including the forearms of small adults and the limbs of young children. Furthermore, the lengths of each of the opposed functional surfaces of the end tabs do not need to be equal so that if, for some particular purpose, a degree of extra security is required, the length of one may be increased as required.

Straps according to the present invention overcome the problem, encountered in prior art straps, in which a releasable and re-attachable securing arrangement employed at the first end of the main portion, has to be moved in order to achieve shortening. Under those circumstances, adjacent areas of strap with large differences in strap thickness either impinge upon the limb or interfere with many ordinary daily living activities. It will be clear that it is possible, though not essential, for straps according to the present invention to be non-releasably secured at the first end of the main portion. This can often be achieved by sewing very close to the securing means at the first anchor point. Such a docking arrangement can virtually eliminate impingement of the secured portion of the strap on the limb in many orthopaedic and orthotic appliances.

Furthermore, if straps according to the present invention are provided with a releasably reattachable first end and the appliance has a plurality of suitable anchor points, such straps may be transferred between anchor points and may be used left or right handed. In addition they may be turned inside out with no loss of utility.

It is, therefore, an object of this invention to provide straps, for use with orthopaedic and orthotic appliances, which have releasable and re-attachable end tabs.

It is another object of this invention to provide straps, in which main strap portions have two outwards facing, parallel, iso-functional surfaces extending over any part which may require to be shortened. End tabs having two portions, both portions being mutually and nonreleasably attached across their respective widths and aligned along their long axes in such a manner that two iso-functional surfaces, co-functional with the two surfaces of the main portion, are generally opposed facing inwards, with an included angle between them and by means of which the end tabs may be kept short, rendering the straps suitable for use in restricted areas,

It is a another object of this invention to provide straps which can be shortened without the need to make adjustments at a releasable and re-attachable first end.

It is yet another object of this invention to provide straps which can be transferred between suitably adapted anchor points on an appliance and which may be used left or right handed or turned inside out and which have an economical length in the main portion.

### BRIEF DESCRIPTION OF THE DRAWINGS

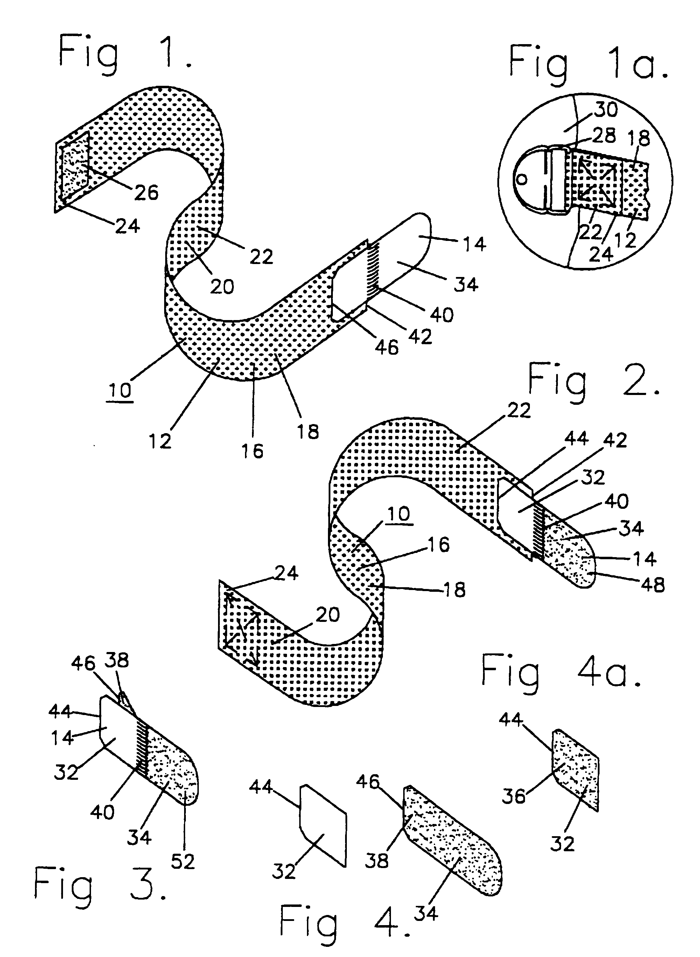

In order that the present invention may be more readily understood the following description and drawings are provided wherein:-
FIG. 1 is a general perspective view of a first side of a first preferred embodiment of a strap according to the present invention;
FIG 1 a is a detail to show how the strap of Fig 1 is anchored at its first end to an orthopaedic or orthotic appliance;
FIG. 2 is a general perspective view of a second side of the strap shown in Fig 1;
FIG. 3 is a general perspective view of an assembled end tab according to a first preferred embodiment of the instant strap;
FIG. 4 is an exploded view of the elements of the end tab shown in Fig 3;
FIG. 4a is a view of the functional surface of one element of the end tab shown in Fig 4;
FIG. 5 is a general perspective view of a first side of a first preferred embodiment of a strap according to the present invention;
FIG. 6 is a general perspective view of a second side of the strap shown in Fig 5;
FIG. 7 is a general perspective view of an assembled end tab according to a second preferred embodiment of the instant strap;
FIG. 8 is an exploded view of the elements of the end tab shown in Fig 3; and
FIG. 8a is a view of the functional surface of one element of the end tab shown in Fig 4.

### DESCRIPTION OF THE INVENTION

### First preferred embodiment

With general reference to FIGS 1 - 4, a strap according to the present invention has the general designation 10 and is intended for use with orthopaedic and orthotic appliances. Strap 10 may be seen to comprise the principal elements of a main portion 12 and a releasably re-attachable end tab 14. Main portion 12 is made from a strip of loop pile fabric with a first side 16 having a first loop pile surface 18 and a second side 20 having a second loop pile surface 22, both loop pile surfaces extending throughout its entire length. Strap 10 has a first end 24 pile surfaces extending throughout its entire length. Strap 10 has a first end 24 adapted by the provision of a piece of hook pile fabric 26 attached to first surface 16. by any standard method including sewing, high frequency welding or even by selecting it to have hook pile on both sides.

As shown by way of example in Fig 1 a, hook pile fabric 26 indicated in hidden detail is used to secure first end 24 of main strap portion 12 to itself around suitable anchor means such as a plastic anchor plate with guide means, represented at 28 and mounted on an appliance 30.

End tab 14 comprises a first portion 32 and a second portion 34 which is longer. Both portions 32 and 34 are made of strips of hook pile fabrics and in this embodiment each has a single hook pile surface 36 and 38, respectively. It will be readily appreciated by those skilled in the art that main portion 12 of strap 10 could equally be made from hook pile fabrics in which case portions 32 and 34 of end tab 14 would be made from loop pile fabric. I prefer to use loop pile fabric for main portion 12 because hook pile is uncomfortable against the skin and strap/skin contact can often occur in orthopaedic and orthotic applications. Portions 32 and 34 of end tab 14 are mutually attached, preferably by strong oversewing, indicated at 40, with hook pile surfaces 36 and 38 opposed facing inwards. Sewing is strongly preferred since high frequency welding can weaken the base material leading to failure under robust traction. This problem is accentuated when functional surfaces rather than flat backings of hook or loop pile narrow fabrics are welded opposed. The attendant loss of performance is not acceptable in orthopaedic and orthotic use.

In this embodiment, 32 and 34 are mutually attached such that their respective ends 44 and 46 are substantially co-linear. Opposed hook pile surfaces 36 and 38 of end tab 14 are separated and then manually pressed against loop pile surfaces 18 and 22 of main strap portion 12 at its second end 42.

The functional lengths of opposed hook pile surfaces 36 of portion 32 and corresponding portion 38 of portion 34 need not exceed the width of the material of end tab 14 in general orthopaedic and orthotic applications and may re-attachable end tab of the present invention may be enjoyed without penalty since it is quite usual to use an overlapped sewn area between the main portion of a strap and a non-releasable end tab of a conventional appliance strap. The length of such an oversewn area will generally equal or exceed the width of material used. The advantage of the instant end tab 14 may be enhanced by making it from strips of fabric somewhat less in width than main portion 12 of strap 10. For instance, if main portion 12 is selected to be of 2" (50mm) material, end tab 14 may be made of 1½'' (38mm) material and in many cases the functional lengths of 36 and 38 may be as little as 1.2" (30mm) whilst still providing excellent attachment characteristics. Such a strategy also improves cosmetic appearance of strap 10. It addition it ensures that in the event of re-attachment of end tab 14 in less than perfect alignment at some point, its edges are less likely to overrun the edges of main portion 12 and this assists smoother running of straps according to the present invention through guide means.

Tabbing end 48 of end tab 14 has a length which need not normally exceed one and a half times the width for 2" (50mm) and 1½'' end tabs according to the present invention but for narrower end tabs a minimum of 2'' (50mm) is preferable. This is not so much for security as for case of handling during tabbing and untabbing.

### Second preferred embodiment

In most important respects, the external appearance and the majority of components of the second preferred embodiment are indistinguishable from the first preferred embodiment.

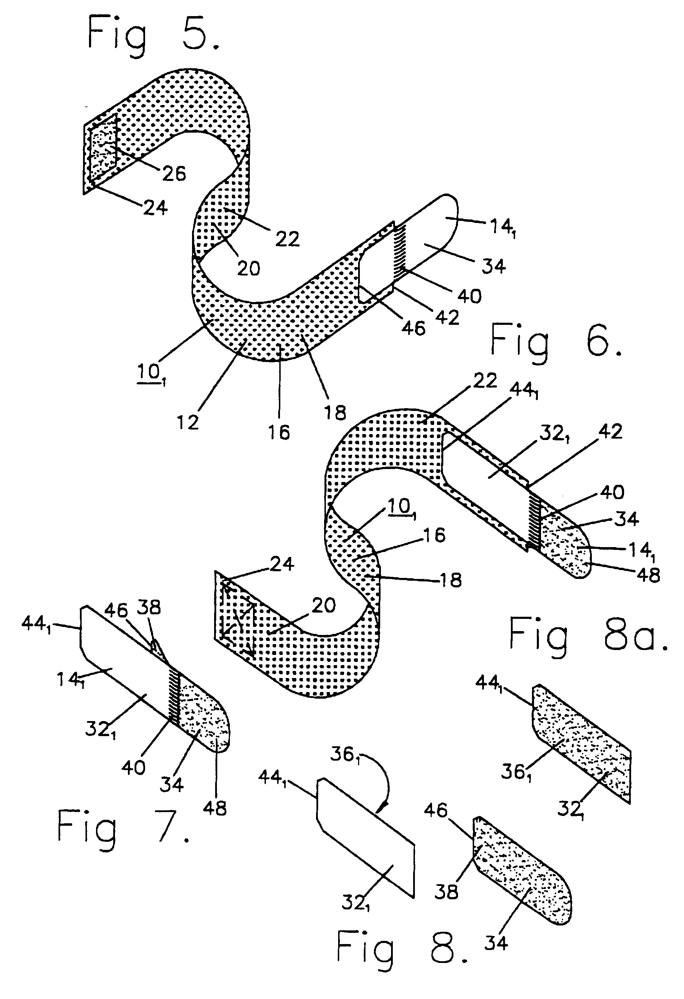

However, as may be seen by reference to FIGS 5 - 8a a strap 10₁ has a main portion 12, substantially similar to that employed in the first embodiment and an end tab 14₁ which is similar in most respects to end tab 14 of the first embodiment. End tab 14₁ has a section 32₁ wherein the length of functional surface 36₁ is at least twice that of 36 of portion 32 in the first embodiment to which it corresponds. Necessarily, the edges 44₁ and 46 of 32₁ and 34, respectively, are not co-linear, however they are substantially parallel. This embodiment would normally be reserved for situations where extra security of attachment beyond normal robust use is required. This might be the case where an appliance is to be fitted on and used by a disturbed or confused patient who might try to interfere with the releasable end tab.

End tab 14₁ of the second embodiment is always used wath functional surface 36₁ of 32₁ attached to the second loop pile surface 22 of main portion 12 of strap 10₁. Furthermore, 36₁ is placed on the side of strap 10₁ towards the patient so that it is effectively hidden and inaccessible when strap 10₁ is secured in use.

Thus, in any given case, with a length ratio of 36₁ to 36 of 2:1, the in line pull-apart tractive load needed to remove end tab 14₁ from main portion 12, will never be less than the equivalent load needed to separate end tab 14 from main portion 12 in the first embodiment, even when surface 38 of portion 34 has been deliberately or inadvertently lifted

It is particularly relevant that the instant straps of both embodiments are intended to be used either circumferentially or hemi-circumferentially about the limbs or trunk. In this mode, forces generated between a strap and the limb will be directed radially. Compressive and inwardly directed radial forces from the strap will be opposed by equal, outwardly directed forces generated in the tissues. During use, this effect also serves to maintain a mutually compressive relationship between hook surface 36 of end tab 14 and hook surface 36₁ of end tab 14₁ against second loop pile surface 22 of main strap portion 12 in strap set ups as shown in Figs 1, 2, 5 and 6, providing excellent security. The second preferred embodiment takes particular advantage of this effect. In addition, tensional forces in the instant straps of both embodiments will be circumferential and will therefore be effectively directed along the hook to loop attachment surfaces between main portion 12 and end tabs 14 and 14₁ which is generally accepted as the optimum arrangement to prevent unwanted separation of such attachments in these materials.

It will now be appreciated that an important advantage of straps according to the present invention and disclosed with reference to the first and second embodiments above, is that they may be shortened quickly and easily without recourse to removal of the appliance from the patient and without unlocking the first end of the strap from the appliance. This is accomplished simply by peeling back the tab end to access the rest of the end tab, peeling this off the main strap portion, which is trimmed as required, then re-attaching the end tab and re-tensioning and tabbing down the strap to itself.

In addition another important advantage of straps of the present invention is that it they may be moved from one suitable anchor point on an appliance to another, for instance to change from left to right handed use. A strap may also be docked at the anchor point with the first end 24 either on view, facing outwards, or obscured, facing inwards, according to choice and circumstances. If a change is made from one choice to the other it is only necessary to remove the end tab turn it over and re-attach it.

Other advantages include the possibility of refurbishing appliances by replacing all, or if circumstances allow, parts of straps.

I contemplate and have made other, less preferred embodiments, including inversions of the two preferred embodiments described above.

From the foregoing it can be realised that this invention provides an improved strap for orthopaedic and orthotic appliances having a reieasable and re-attachable end tab of sewn construction which provides for easier shortening and adjustment and enhanced general versatility. Whilst this invention has been described in respect of two preferred embodiments, modifications and adapations may readily be made by those skilled in the art of orthopaedics and orthotics and it is intended that the claims cover any such modifications and adapations which fall within the spirit and scope of the invention.

### The invention comprises

1. Strap means for an orthopaedic or orthotic appliance made from strips of loop pile and hook pile fabrics wherein
   a first main portion has a first end provided with means for attachment to first anchoring means on an appliance said main portion also having first and second isofunctional surfaces co-functional with a second portion in the form of an end tab which comprises releasable and re-attachable means for securing said second portion to said first portion at a second end and said second portion also provides means for tabbing said end tab to said main portion of said strap during use.
   the second end tab portion has first and second elements mutually and nonreleasably attached by sewing across their respective widths with iso-functional surfaces opposed facing inwards and edges substantially parallel. The opposed functional surfaces of the first and second elements of the end tab portion provide means for releasable reattachment of the end tab to the main portion of the strap
   the second element of the end tab has a further area of functional surface which is not coextensive with the first element and provides tab means for securing the second portion to the first portion of the strap during use.
2. Strap means for an orthopaedic or orthotic appliance made from loop pile and hook pile strips comprising a first main portion having a first end provided with meansfor attachment to first anchoring means on an appliance, said main portion having first and second iso-functional surfaces and a second portion which comprises and re-attachable means for securing said second portion tosaid first portion at a second end of said main portion, said second portion providing tab means for securing said second portion to said main portion of said strap during use; the second portion having first and second elements connected to each other providing opposed iso-functional surfaces, said opposed surfaces of the first and second elements providing means for releasable reattachment of the second portion to the main portion of the strap.

## Claims

1. Strap means for an orthopaedic or orthotic appliance, the strap means being made from loop pile and hook pile strips and comprising: a first main portion (12) having a first end (24) provided with means for attachment to the appliance, said main portion having first and second iso-functional surfaces (18,22); and a second portion which comprises releasable and re-attachable connecting means (14) for securing said second portion to said main portion at a second end of said main portion, said second portion providing tab means for securing said second portion to a further area of said main portion of said strap during use of the appliance; the second portion having first and second elements (32,34) connected to each other by sewing across their widths to provide opposed iso-functional surfaces (36,38), said opposed surfaces of the first and second elements providing said connecting means for releasable re-attachment of the second portion to the second end of the main portion of the strap, wherein the width of said second portion is in the range 0.7 to 1.0 times the width of the main portion.

2. Strap means according to claim 1, wherein the length of one at least of said opposed surfaces of the first and second elements (32,34) is in the range 0.7 to 1.5 times the width of the second portion.

3. Strap means according to claim 1 or 2, wherein the length of said tab means is in the range 1 to 1.7 times the width of the second portion but is not less than about 50 mm.

4. Strap means according to any of claims 1 to 3, wherein the main portion is made from loop pile fabric and the first and second elements of the second portion are made from hook pile fabric.

5. Strap means according to any preceding claim, wherein the first end of the main portion is provided with means for attachment to an anchoring means on the appliance.

6. Strap means according to claim 5 when dependent on claim 4, wherein the attachment means comprises a patch of hook pile fabric attached at or near the first end of the main portion.

7. Strap means according to any preceding claim, wherein said first element of said second portion has an inwardly directed functional surface and said second element of said second portion also has an inwardly directed functional surface, the first and second elements being connected together such that a section of the length of the inwardly directed functional surface of one of the first and second elements is exposed to provide said tab means.

8. Strap means according to claim 7, wherein said first and second elements are substantially the same length.

9. Strap means according to claim 7, wherein one of said first and second elements has a length substantially greater than that of the other of said first and second elements.

10. Strap means for an orthopaedic or orthotic appliance made from strips of loop pile and hook pile fabrics, wherein a first man portion has a first end provided with means for attachment to first anchoring means on an appliance, said main portion also having first and second iso-functional surfaces co-functional with a second portion in the form of an end tab which comprises releasable and re-attachable means for securing said second portion to said first portion at a second end, and said second portion also provides means for tabbing said end tab to said main portion of said strap during use; the second end tab portion having first and second elements mutually and non-releasably attached by sewing across their respective widths with iso-functional surfaces opposed facing inwards and edges substantially parallel; the opposed functional surfaces of the first and second elements of the end tab portion providing means for releasable re-attachment of the end tab to the main portion of the strap, the second element of the end tab having a further area of functional surface which is not co-extensive with the first element and provides tab means for securing the second portion to the first portion of the strap during use.
